# EUROPEAN PATENT SPECIFICATION

(11) **EP 3 117 217 B1**
(45) Date of publication and mention of the grant of the patent: **18.07.2018**
(21) Application number: 15716745.3
(22) Date of filing: 10.03.2015
(51) Int. Cl.: G01N 33/58, G01N 33/68, G01N 33/90

(54) **ANALYTICAL METHOD FOR THE IDENTIFICATION OF AT LEAST ONE GLYCOFORM OF THE TRANSFERRIN PROTEIN**
ANALYTISCHES VERFAHREN ZUR IDENTIFIZIERUNG VON MINDESTENS EINER GLYCOFORM DES TRANSFERRINPROTEINS
PROCÉDÉ ANALYTIQUE POUR L'IDENTIFICATION D'AU MOINS UNE GLYCOFORME DE LA PROTÉINE DE TRANSFERRINE

(30) Priority: 12.03.2014 IT MI20140395
(43) Date of publication of application: 18.01.2017
(73) Proprietor: Università Degli Studi Di Verona, 37129 Verona (IT)
(72) Inventor: DE PALO, Elio Franco, I-37136 Verona (IT); TAGLIARO, Franco, I-37131 Verona (IT); SORIO, Daniela, I-37137 Verona (IT); BORTOLOTTI, Federica, I-37122 Verona (IT)
(74) Representative: Zambardino, Umberto
(86) International application number: PCT/EP2015/054896
(87) International publication number: WO 2015/135900

(56) References cited:
- WO-A1-95/04932
- J. P. BERGSTROM ET AL: "Clinical Characteristics of Carbohydrate-Deficient Transferrin (%Disialotransferrin) Measured by HPLC: Sensitivity, Specificity, Gender Effects, and Relationship with other Alcohol Biomarkers", ALCOHOL AND ALCOHOLISM, vol. 43, no. 4, 14 February 2008 (2008-02-14), pages 436-441, XP055151798, ISSN: 0735-0414, DOI: 10.1093/alcalc/agn017
- SIMON M. YEH ET AL: "Characterization of transferrin metal-binding sites by diffusion-enhanced energy transfer", BIOCHEMISTRY, vol. 19, no. 22, 1 October 1980 (1980-10-01), pages 5057-5062, XP055152003, ISSN: 0006-2960, DOI: 10.1021/bi00563a019
- YOSHINORI YAMAGUCHI ET AL: "Sodium Dodecyl Sulfate Polyacrylamide Slab Gel Electrophoresis and Hydroxyethyl Cellurose Gel Capillary Electrophoresis of Luminescent Lanthanide Chelate-labeled Proteins with Time-Resolved Detection", ANALYTICAL SCIENCES, vol. 25, no. 3, 1 March 2009 (2009-03-01), pages 327-332, XP055152005, ISSN: 0910-6340, DOI: 10.2116/analsci.25.327
- ANUMULA ET AL: "Advances in fluorescence derivatization methods for high-performance liquid chromatographic analysis of glycoprotein carbohydrates", ANALYTICAL BIOCHEMISTRY, ACADEMIC PRESS INC, NEW YORK, vol. 350, no. 1, 1 March 2006 (2006-03-01) , pages 1-23, XP024942466, ISSN: 0003-2697, DOI: 10.1016/J.AB.2005.09.037 [retrieved on 2006-03-01]
- BORTOLOTTI F ET AL: "Carbohydrate-deficient transferrin (CDT) as a marker of alcohol abuse: A critical review of the literature 2001-2005", JOURNAL OF CHROMATOGRAPHY B: BIOMEDICAL SCIENCES & APPLICATIONS, ELSEVIER, AMSTERDAM, NL, vol. 841, no. 1-2, 1 September 2006 (2006-09-01), pages 96-109, XP027981921, ISSN: 1570-0232 [retrieved on 2006-09-01]

## Description

The present invention generally refers to an analytical method for the identification of at least one transferrin glycoform and/or isoform, particularly regarding carbohydrate-deficient transferrin (CDT), possibly present in a complex biological matrix, by contact (functionalisation) of the latter with a source of lanthanide 3+ ion. The formed glycoform-lanthanide ion adduct can be identified through known analytical techniques, preferably with fluorescence detection.

### State of the art

Transferrin is a glycoprotein having the function of transporting Iron in the human organism. Its structure has two C- and N- terminal domains each of which are capable of bonding an Fe3+ ion. Thus, each transferrin molecule is capable of bonding two iron ions, balancing the charge with an anion (synergic anion, usually carbonate) typically depending on the pH. In clinically healthy ("normal") people, the iron saturation level in human serum is about 30%, this meaning that the protein still has a percentage of free sites, potentially capable of interacting with 3+ ions.

Transferrin, in its various isoforms, is characterised by a high microheterogeneity both of amino acid and glycosylation sequence as well as sialic groups. In particular, in its structure, the transferrin molecule comprises glycan chains, each of which usually terminates with a sialic acid residue. Theoretically, transferrins with sialic acid residue from 0 up to 8 (with isoelectric points (pI) comprised between 5.1 and 5.9) are possible. However, the main glycoform/sialoform in human serum (65-80%), is tetrasialo-transferrin, i.e. having four glycan chains, thus four sialic acid residues and having a 5.4 pI (for general reference see for example Schellenberg et al., Clin Chem Lab Med 2013, 51(5), 991-996).

There are transferrin glycoforms revealing deficiencies in the glucidic and sialic acid chains, and which can be used as a diagnostic marker. Said glycoforms (generally indicated as CDT) were identified as: asialo-, mono- and di-asialo transferrin, respectively containing 0, 1 and 2 sialic acid residues. In addition, these glycoforms are characterised by pI values comprised between 5.7 and 5.9.

Alterations of said forms were observed in congenital neurological disorders attributed to the protein glycosylation deficiency (CDGS - carbohydrate deficient glycosylation syndromes). Even during pregnancy there was observed a slight increase of carbohydrate deficient transferrins. When diagnosing abuse of alcohol, the use of some of these glycoforms as biochemical markers is widely known. In particular, the increase of the disialo component in abuse of alcohol was attributed both to glycosylation defects and an increase of the plasmatic and membrane desialization processes, which would lead to a partial desialization of transferrin (see for example van Eijk et al. Clin Chim Acta, 132, (1983), 167-171).

Thus, analytical methods for the qualitative/quantitative determination of the amount of transferrin glycoforms, with particular reference to the CDT isoforms, as herein defined in detail, were developed and optimised in the clinical and toxicological diagnostic field.

US4626355 and WO95/04932 describe a method for the analytical separation of some CDTs by separation on ion exchange solid phase, under controlled pH conditions, so as to hold some glycoforms in the column, allowing the elution of the asialo, mono- and disialo forms.

US5432059 discloses a method for the detection of CDT glycoforms by re-glycosylation of the protein with a fluorescent glycoprotein, in the presence of suitable reagents and enzymes.

In addition, CDT detection methods comprising the use of antibodies as biological markers, capable of selectively bonding to CDT, thus allowing the subsequent analysis thereof, are known.

EP1378521 describes a method comprising the use of antibodies which - in an aqueous environment- are capable of binding to CDT, characterised in that it is possible to avoid the use of the solid phases, as it generally occurs for this type of modifications (for example see GB9827411).

There is therefore the need to find a method that is easy to apply, that can be suitable for different types of complex biological matrices, and sensitive enough to allow detecting even slight variations of the amount of transferrin glycoforms, with particular reference to carbohydrate deficient transferrins, especially the disialo-transferrin. Basis of the present invention is the finding that it is possible to mark the transferrin possibly present in a complex biological matrix, with a lanthanide 3+ ion, thus obtaining a functionalised protein (transferrin-lanthanide 3+ ion adduct) which can be separated in its glycoforms and detected using analytical techniques known in the art. The present method allows obtaining high sensitiveness, allowing in addition to identify and measure the various transferrin glycoforms, including CDT, both from a quality and quantity point of view, providing major indications on the clinical condition of the patient, also including possible abuse of alcohol.

### Summary of the invention

The invention is set out in the appended claims.

### Description of the figures

Figure 1: HPLC chromatogram in fluorescence mode (exc 298, em 550 nm - mV vs min) of human serum with high value of derivatised disialo-transferrin.
Figure 2: CE electropherogram in fluorescence mode (exc 280, em >460 nm - RFU vs min) of derivatised human serum.
Figure 3: HPLC chromatogram in fluorescence mode (exc 298, em 550 nm - mV vs min) of derivatised human serum.

### Detailed description

The term "analytical separation and detection" is intended to indicate that the transferrin glycoforms functionalised with lanthanide 3+ ion, are identified by qualitative separation, for example by chromatography according to their elution times, and thus detected and measured by analytical detectors.
"Quantitative separation" means that the transferrin glycoforms are also separated and isolated upon identification.
Unless otherwise specified, the term "CDT" indicates the carbohydrate-deficient transferrin protein in the glycoforms: asialo, mono- and diasialo forms, possibly present in the analysed biological matrix.
As previously mentioned, said glycoforms have a number of sialic acid residues below 3 and pI greater or equal to 5.7. They are due to the absence of one or more sialic acid residues, which are instead present in the transfeferrin protein under "normal" physiological conditions.

The expression "marked or functionalised glycoform" is intended to indicate the involved glycoform transferrin (including carbohydrate deficient transferrins), functionalised with a lanthanide 3+ ion, as hereinafter described in detail. In practice, due to the contact between the biological matrix and source of lanthanide ions, the free Fe3+ sites of the protein are occupied, even up to saturation, by the selected lanthanide ion, leading to the formation of the protein-lanthanide adduct, preferably CDT-lanthanide 3+ ion adduct.

As previously mentioned, the invention refers to a method useful for the identification of one or more transferrin glycoforms and/or isoforms and/or sialoforms and in particular CDTs in a complex biological matrix, comprising the contact of the latter with a source of lanthanide 3+ ions (step a.), the formation of the protein-lanthanide 3+ ion adduct, (step b.) and the subsequent analytical separation and detection of at least one marked lanthanide glycoform (step c.). Advantageously, the present method also allows measuring minimum percentage variations of the various transferrin glycoforms, in a simple, accurate and reproducible fashion.

The biological matrix of the present method is preferably a human or animal body fluid, the human body fluid being particularly preferred. More preferably, the biological matrix is selected from: blood, plasma, vitreous humour, saliva and, preferably blood serum or cerebrospinal fluid (liquor).

In an embodiment, the source of lanthanide 3+ ions comprises an organic or, preferably, inorganic salt of a 3+ ion of an element selected from among: europium (Eu), terbium (Tb), erbium (Er), praseodymium (Pr), ytterbium (Yb), preferably terbium.

Preferred salts are selected from: chlorides, nitrates, acetylacetonates, nitriloacetates (NTA) and acetates, chloride salt being particularly preferred.

Generally, the source of lanthanide 3+ ion is an organic or, preferably, aqueous solution, comprising the salt of a lanthanide metal as mentioned above. In case of organic solutions, these solutions are prepared by dissolving the salt in an organic solvent selected from: acetonitrile, tetrahydrofuran and, preferably, alcohols with 1 to 4 carbon atoms, methanol being particularly preferred. Preferred aqueous solutions are obtained by dissolving the salt in a saline solution, preferably in water.

The lanthanide ion solutions that can be used in the present method may have final concentrations comprised between 0.1 micromols/L and 1mM for example selected according to the type of fluid or ion used.

Before the contact step a., it may be convenient to dilute the selected biological matrix with a diluting solution, such as for example, water or saline solution. Generally, the matrix is diluted at ratios comprised between 1:2 and 1:10, depending on the type of analytical technique intended to be used and/or depending on the composition and chemical/physical characteristics of the matrix. This dilution is useful for optimising the analysis considering the quenching effect, the saturation of the amount of samples used, and also safeguarding the utilized analytical systems. Possibly, the sample may also be added with a suitable stabilising agent mainly having the function of chelating the free iron, i.e. not bound to the protein. The stabilising agent may be added during the preparation of the sample or even directly into the elution phase, in case of HPLC analysis. In any case, the presence of the stabiliser allows obtaining a better signal, thus guaranteeing solutions of the sample with greater stability, in that it considerably reduces the formation of the derived hydroxide of the selected lanthanide, which could otherwise be formed. Suitable chelating agents may be selected from: EDTA, deferoxamine B (FeDFO, for example sold under the name Desferal, mesylate salt), and the like. The contact according to step a. may occur at room temperature (approximately comprised between 15° C and 40° C), for example by mixing a human body fluid, possibly diluted, and an aqueous solution containing terbium chloride, in the presence of deferoxamine B.

The mixture is left to rest for an incubation period, so as to allow the functionalisation of the transferrin glycoforms, with lanthanide 3+ ion, according to step b. Said incubation period allows the formation of the protein-lanthanide 3+ ion adduct, preferably by saturating the free Fe3+ binding sites, and it can also be carried out within very short periods of time, i.e. in the order of 4 or 5 minutes.

It is noted that the adduct formed in step b., is stable over time and it can also be preserved for several days, without substantially interfering with the analysis and composition of the treated body fluid. Possibly, at the end of step b., it is possible to carry out an intermediate solid/liquid separation step, for example by centrifugation, generally carried out when the sample reveals impurities or opalescence which could affect the ease of execution of the test, or somehow jeopardise the sensitiveness of the method.

The sample containing the transferrin-lanthanide 3+ ion adduct is thus subjected to analytical separation and detection according to step c.

Preferably, in step c. the asialo, mono- and disialo-transferrin glycoforms (i.e. carbohydrate deficient transferrins herein indicated with CDT) are identified, the disialo glycoform being particularly preferred.

To this regard, there can be advantageously used analytical techniques and instruments known to the skilled in the art, for example selected according to the type of used matrix, or the available amount of sample (for a general reference see Biochimica Clinica, 2006, Vol 30, n.3 pages 203-209). Preferably, the step c. of the present method comprises the analytical separation by HPLC or capillary electrophoresis (CE). In case of HPLC separation, the eluent phase may contain a suitable compound useful as an ionic pair agent for secondary negative charge, thus allowing a better resolution of the final signal. Preferably, said compound is 1,4-diaminobutane (DAB). In case of CE separation, the running buffer may contain a coating compound for reducing the ion interactions between the walls of the capillary and the ionic analyte to be separated, preferably said compound is DAB.

By way of non-limiting example, the analytical separation of one or more glycoforms of the CDT-lanthanide 3+ ion adduct according to the present method may occur by using a weak anion exchange HPLC column, eluting in concentration gradient with a buffer solution with pH for example comprised between 6 and 9, in the presence of possible stabilisers or sequestering agents. Still by way of example, in the case of CE analysis, there can be used capillaries with a 25-50 microns diameter, with length comprised between 20-100 cm, using a borate buffer or the like, possibly containing traces of the lanthanide 3+ ion salt, preferably at amounts comprised between 0.1 and 1000 micromols/L.

In an equally preferred embodiment, the step c. comprises the analytical detection and measurement of glycoforms of transferrin by fluorescence.

The use of a fluorescence detector, with suitable excitation and emission wavelengths, is particularly convenient in that it allows obtaining optimal detection sensitiveness and specificity, capable of measuring even slight percentage variations of the various transferrin glycoforms, including CDTs.

Typical final conditions of the fluorescence detectors used may be excitation (ex) comprised between 250-320 nm and emission (em) greater or equal to 400 nm for HPLC and CE.

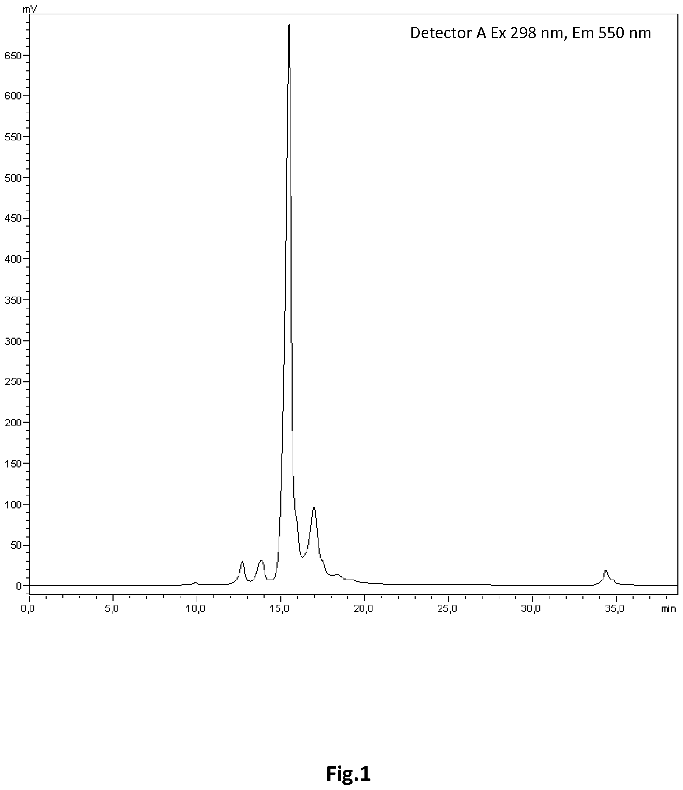

To this extent, figure 1 shows a HPLC chromatogram, of a human serum treated with a terbium chloride aqueous solution obtained with ex298 em550 nm fluorescence detection. The signals of disialo- (approximately 13 min), trisialo- (approximately 14 min); tetrasialo- (approximately 15-16min) and pentasialo-transferrin (between 17 and 18 min) can be observed.

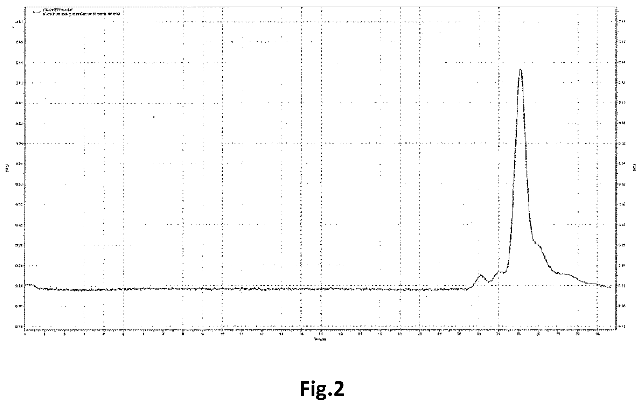

Figure 2, shows a fluorescence electropherogram of a serum sample treated with a terbium chloride aqueous solution, in which the di-, trisialo carbohydrate deficient signals (at 23 and 24 min respectively) as well as tetra-, and penta-sialo transferrin forms (from 24.5 to about 26 min) can be identified.

As observable from figures 1-2, the presence of high amounts of tetrasialo-transferrin may be used as internal standard in the elution/migration times, with the aim of attaining a more accurate identification of the other components by measuring the relative times.

Besides high sensitiveness, the present method allows obtaining plots (chromatograms/electropherograms) in which the interference effects, caused by the type of the analysed biological matrix, are entirely marginal, contrary to what observed in the prior art methods. Regarding this, see for example figure 2, in which it can be noted the absence of interfering signals, macroscopically evident in the analysis executed using conventional methods through UV detection. This suggests that the present method is also potentially applicable to biological matrices different from human serum or blood in general, however guaranteeing optimal results both in terms of clarity and sensitiveness.

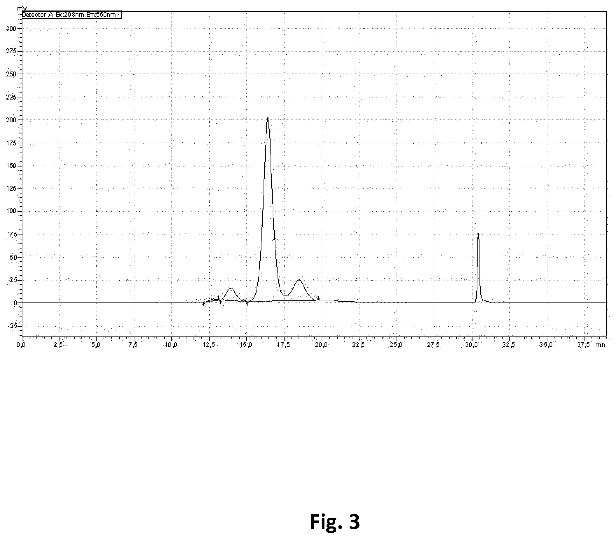

Measuring the chromatographic peak areas advantageously allows calculating the percentages of the detected glycoforms, and thus obtaining major information on the clinical/toxicological picture of the individual subject of study, for example with respect to the values obtained using the same biological matrix in healthy reference subjects. In practice, the values regarding a glycoform, measured using the present method, correspond to the percentage value of the relative peak, with respect to the amount of total protein. For example, as illustrated in figure 3, considering the disialo areas (56786, at 12.7 min), trisialo (592805, at 13.9 min) and tetra+pentasialo (10487682, at 16.4 min), the percentage of calculated disialo glycoform is 0.51% of the total area.

In an additional embodiment, the present method may comprise a possible step for the quantitative separation d., for example of one or more CDT glycoforms and/or isoforms, typically by methods known to the skilled in the art, such as for example, preparative columns and the like.

In a further aspect, the invention refers to the use of the present method for the identification of possible diseases or clinical conditions associated to the presence of one or more glycoforms, preferably CDT, in particular the disialo glycoform. When the biological matrix in question is human, the present method is suitable for diagnosing a condition of addiction or abuse of alcohol of the person subject of the analysis.

Thus, the invention also refers to a diagnostic method for the detection of diseases or clinical conditions associated to the presence of at least one transferrin glycoform and/or isoform in a living being, preferably human, comprising:
- collecting a human fluid sample, selected as above indicated;
- contact of said sample with a source of lanthanide 3+ ion, as above indicated;
- analytical separation and detection of at least one transferrin glycoform and/or isoform as above indicated;
- possible comparison of the obtained values with values measured in healthy reference subjects.

The expression "healthy subjects" is intended to indicate human beings having carbohydrate deficient glycoforms, and in particular disialo glycoforms, that are normal and not associable to altered clinical/toxicological conditions.

Besides high sensitivity, the present method is also characterised by the ease of execution in that no particular reagents or preliminary treatments of the sample being analyzed are required. In addition, the possibility of utilising analytical techniques with fluorescence detection also allows analysing limited amounts of biological sample, guaranteeing high reliability of the method. Actually, in case of alleged positive results it is not necessary to carry out a confirmation test as it is instead required in some prior art diagnostic methods.

A further advantage lies in the fact that the present method may also be adapted to different types of complex biological matrices, making the invention extremely versatile and with wide applicability.

The invention will be now described with the following experimental part, meant for illustrative and non-limiting purposes.

### EXPERIMENTAL PART

### Example 1.1: preparation of the transferrin-lanthanide 3+ ion adduct according to the method of the invention.

Sample: 40 microls of serum sample (or standard Apo at the conc. of about 10 micromols/L or control in use with the conventional methods) to which there were added 8 microls of an aqueous saline solution of the lanthanide chloride salt (final concentration of about 500 micromols/l), subsequent addition of 15 microls of Deferoxamine-B stabiliser (FeDFO, for example sold under the name Desferal, mesylate salt) and addition of water for a final volume of 150 microls.

### Example 1.2: detection of the adduct of the example 1.1 by HPLC (figure 1).

The column used is a WAX column (weak anion exchange CLINREP) 4.6x5 mmxcm with specific guard column and 0.22 microm filter and thermostatation at 50°C. 1 ml/min flow. Tris Buffer 20 mM mobile phase at pH comprised between about 7 and 9, with addition of NaCl 0.5 M and DAB (1,4 diaminobutane) 18 mM. 0 to 20% gradient of phase B. Phase A also contains the Deferoxamine-B stabiliser (FeDFO, desferal) at the concentration of 50-100 micromols/L.

At the end of the run, the column is regenerated using a 2 M sodium chloride solution. Total duration of the analysis about 40 mins.

These conditions were tested on a human serum sample treated with terbium chloride and the chromatogram is indicated in figure 1.

### Example 1.3: detection of the adduct of example 1.1. through CE analysis (figure 2).

Preliminary data revealed the di-, tri-, tetra- and penta-sialo transferrin isoforms. The fluorescence detector in use is of the LED type. The executed tests used capillaries with a diameter of 50 microns, length of about 50-70 cm. The running buffer is a borate buffer, pH 7-9, 120 mM containing DAB 6 mM, separations executed with ddp of 30 kV. The sample is injected under pressure (0.5 psi) and the separation run lasts about 30 mins. The treatment of the sample is entirely similar to what is provided for the HPLC of example 1.2, i.e.: serum (standard or control), addition of Lanthanide saline solution (final 500 micromols/L in the injected sample).

Figure 2 indicates the CE spectrum obtained using a serum sample treated with terbium chloride.

## Claims

1. Method for the identification of at least one glycoform and/or isoform of transferrin possibly present in a complex biological matrix, said method comprising the steps of:
a. contact a sample of said matrix with a source of lanthanide 3+ ion, wherein the source of lanthanide 3+ ion is an organic or aqueous solution comprising a salt of a +3 ion of an element selected from europium, erbium, praseodymium, ytterbium and, preferably, terbium, obtaining a mixture;
b. leaving said mixture to rest for an incubation period so as to allow the functionalisation, with lanthanide 3+ ion, of at least one transferrin glycoform and/or isoform, forming the transferrin-lanthanide 3+ ion adduct;
c. analytical separation by high performance liquid chromatography (HPLC) or capillary electrophoresis (CE) analysis and measurement and detection by fluorescence of said at least one functionalised glycoform and/or isoform.

2. Method according to claim 1 for the identification, and possibly measurement, of at least one transferrin glycoform, preferably carbohydrate-deficient transferrin, selected from: asialo-, monosialo-, disialo- transferrin.

3. Method according to claim 2, for the identification and measurement of the disialo-transferrin.

4. Method according to claims 1-3, wherein said biological matrix is an animal or, preferably, a human body fluid.

5. Method according to claim 4, wherein said fluid is selected from among: plasma, blood, vitreous humour, saliva and preferably, blood serum or cerebrospinal fluid.

6. Method according to the preceding claims, wherein said source of lanthanide 3+ ions is an organic or aqueous solution of a lanthanide 3+ salt selected from: nitrates, acetates, acetylacetonates, nitriloacetates and preferably chlorides.

7. Method according to claim 6, wherein said source is an aqueous solution obtained by dissolving the salt of the lanthanide ion in a saline solution.

8. Method according to claim 6, wherein said source is an organic solution obtained by dissolving the salt of the lanthanide ion in an organic solvent selected from: acetonitrile, tetrahydrofuran, alcohols with 1 to 4 carbon atoms, preferably methanol.

9. Method according to the preceding claims, wherein the functionalisation according to step b. occurs by saturating the free Fe3+ binding sites of at least one transferrin glycoform, preferably carbohydrate-deficient transferrin, with a lanthanide 3+ ion.

10. Method according to the preceding claims, further comprising a step d. for the quantitative separation of at least one glycoform detected in step c.

11. Method according to the preceding claims, for the determination of a clinical/toxicological state associated to the presence of at least one transferrin glycoform, preferably carbohydrate-deficient transferrin.

12. Method according to claim 11, for the detection of an alcohol abuse or addiction condition in a human being.

## Patentansprüche

1. Verfahren zur Identifikation von wenigstens einer Glykoform und/oder Isoform von Transferrin, die möglicherweise in einer komplexen biologischen Matrix vorhanden sind, wobei das Verfahren die Schritte umfasst:
a. Kontaktieren einer Probe der genannten Matrix mit einer Quelle für ein Lanthanid 3+ Ion, wobei die Quelle für das Lanthanid 3+ Ion eine organische oder wässrige Lösung ist, die ein Salz eines 3+ Ions eines Elements enthält, das ausgewählt ist aus Europium, Erbium, Praseodym, Ytterbium sowie vorzugsweise Terbium, so dass eine Mischung erhalten wird;
b. Ruhenlassen der Mischung für einen Inkubationszeitraum, um die Funktionalisierung von wenigstens einer Transferrin-Glykoform und/oder Isoform mit einem Lanthanid 3+ Ion zu ermöglichen, so dass das Transferrin-Lanthanid 3+ Ion-Addukt gebildet wird;
c. Analytische Trennung mittels Hochleistungsflüssigkeitschromatographie (HPLC)- oder Kapillarelektrophorese (CE)-Analyse und Messung und Nachweis der genannten wenigstens einen funktionalisierten Glykoform und/oder Isoform mittels Fluoreszenz.

2. Verfahren nach Anspruch 1 für die Identifikation sowie möglicherweise Messung von wenigstens einer Transferrin-Glykoform, vorzugsweise eines kohlenhydratarmen Transferrins, die ausgewählt ist aus Asialo-, Monosialo-, Disialo-Transferrin.

3. Verfahren nach Anspruch 2 zur Identifikation und Messung des Disialo-Transferrins.

4. Verfahren nach den Ansprüchen 1 bis 3, wobei die genannte biologische Matrix eine tierische oder vorzugsweise eine humane Körperflüssigkeit ist.

5. Verfahren nach Anspruch 4, wobei diese Flüssigkeit ausgewählt ist aus Plasma, Blut, Glaskörperflüssigkeit, Speichel und vorzugsweise Blutserum oder Cerebrospinalflüssigkeit.

6. Verfahren nach den vorausgehenden Ansprüchen, wobei die genannte Quelle für Lanthanid 3+ Ionen eine organische oder wässrige Lösung eines Lanthanid 3+ Salzes ist, das ausgewählt ist aus Nitraten, Acetaten, Acetylacetonaten, Nitrilacetaten und vorzugsweise Chloriden.

7. Verfahren nach Anspruch 6, wobei die genannte Quelle eine wässrige Lösung ist, die erhalten wird durch Auflösen des Salzes des Lanthanid-Ions in einer Salzlösung.

8. Verfahren nach Anspruch 6, wobei die genannte Quelle eine organische Lösung ist, die erhalten wird durch Auflösen des Salzes des Lanthanid-Ions in einem organischen Lösemittel, das ausgewählt ist aus Acetonitril, Tetrahydrofuran, Alkoholen mit 1 bis 4 Kohlenstoffatomen, vorzugsweise Methanol.

9. Verfahren nach den vorausgehenden Ansprüchen, wobei die Funktionalisierung gemäß Stufe b. als Sättigung der freien Fe3+-Bindungsstellen wenigstens einer Transferrin-Glykoform, vorzugsweise eines kohlenhydratarmen Transferrins, mit einem Lanthanid 3+ Ion erfolgt.

10. Verfahren nach den vorausgehenden Ansprüchen, das außerdem eine Stufe d. für die quantitative Abtrennung wenigstens einer Glykoform umfasst, die in Stufe c. nachgewiesen wurde.

11. Verfahren nach den vorausgehenden Ansprüchen zur Bestimmung eines klinischen/toxikologischen Zustands, der mit der Gegenwart von wenigstens einer Transferrin-Glykoform, vorzugsweise eines kohlenhydratarmen Transferrins, assoziiert ist.

12. Verfahren nach Anspruch 11 zum Nachweis eines Alkoholmissbrauchs oder einer Suchterkrankung bei einem menschlichen Wesen.

## Revendications

1. Procédé d'identification d'au moins une glycoforme et/ou une isoforme de la transferrine éventuellement présentes dans une matrice biologique complexe, ledit procédé comprenant les étapes consistant à :
a. mettre en contact un échantillon de ladite matrice avec une source d'ion de lanthanide ³⁺, dans lequel la source d'ion de lanthanide ³⁺ est une solution organique ou aqueuse comprenant un sel d'un ion ³⁺ d'un élément sélectionné parmi l'europium, l'erbium, le praséodyme, l'ytterbium et, de préférence, le terbium, obtenant un mélange ;
b. laisser ledit mélange au repos pendant une période d'incubation de manière à permettre la fonctionnalisation, avec un ion de lanthanide ³⁺, d'au moins une glycoforme et/ou une isoforme de transferrine, formant l'adduit de transferrine-ion de lanthanide 3⁺ ;
c. réaliser la séparation analytique par analyse de chromatographie liquide à hautes performances (CLHP) ou électrophorèse capillaire (EC) et effectuer la mesure et la détection par fluorescence desdites au moins une glycoforme et/ou isoforme fonctionnalisées.

2. Procédé selon la revendication 1 d'identification, et éventuellement de mesure, d'au moins une glycoforme de la transferrine, de préférence la transferrine déficiente en glucides, sélectionnée parmi : l'asialo-transferrine, la monosialo-transferrine, la disialo-transferrine.

3. Procédé selon la revendication 2 d'identification et de mesure de la disialo-transferrine.

4. Procédé selon les revendications 1 à 3, dans lequel ladite matrice biologique est un fluide corporel animal ou, de préférence, humain.

5. Procédé selon la revendication 4, dans lequel ledit fluide est sélectionné parmi : le plasma, le sang, le corps vitré, la salive et, de préférence, le sérum sanguin ou le liquide céphalo-rachidien.

6. Procédé selon les revendications précédentes, dans lequel ladite source d'ions de lanthanide ³⁺ est une solution organique ou aqueuse de sel de lanthanide ³⁺ sélectionné parmi : les nitrates, les acétates, les acétylacétonates, les nitriloacétates et de préférence les chlorures.

7. Procédé selon la revendication 6, dans lequel ladite source est une solution aqueuse obtenue en dissolvant le sel de l'ion de lanthanide dans une solution saline.

8. Procédé selon la revendication 6, dans lequel ladite source est une solution organique obtenue en dissolvant le sel de l'ion de lanthanide dans un solvant organique sélectionné parmi : l'acétonitrile, le tétrahydrofurane, des alcools ayant 1 à 4 atomes de carbone, de préférence le méthanol.

9. Procédé selon les revendications précédentes, dans lequel la fonctionnalisation selon l'étape b. se produit en saturant les sites de liaison Fe³⁺ libres d'au moins une glycoforme de la transferrine, de préférence de la transferrine déficiente en glucides, avec un ion de lanthanide ³⁺.

10. Procédé selon les revendications précédentes, comprenant en outre une étape d. de séparation quantitative d'au moins une glycoforme détectée à l'étape c.

11. Procédé selon les revendications précédentes de détermination d'un état clinique/toxicologique associé à la présence d'au moins une glycoforme de la transferrine, de préférence la transferrine déficiente en glucides.

12. Procédé selon la revendication 11 de détection d'une consommation abusive d'alcool ou d'une condition de dépendance chez un être humain.
